# EUROPEAN PATENT SPECIFICATION

(11) **EP 2 900 836 B1**
(45) Date of publication and mention of the grant of the patent: **10.05.2023**
(21) Application number: 13840845.5
(22) Date of filing: 25.09.2013
(51) Int. Cl.: C12Q 1/6853, C12N 15/11

(54) **TWO-PRIMER PCR FOR MICRORNA MULTIPLEX ASSAY**
PCR MIT ZWEI PRIMERN FÜR EIN MIKRORNS-MULTIPLEX-ASSAY
PCR À DEUX AMORCES POUR DOSAGE MULTIPLEXE DE MICROARN

(30) Priority: 28.09.2012 US 201261707670 P
(43) Date of publication of application: 05.08.2015
(73) Proprietor: Cepheid, Sunnyvale, CA 94089 (US)
(72) Inventor: HIGUCHI, Russell, Sunnyvale California 94089 (US); LAI, Edwin Wei-Lung, Sunnyvale California 94089 (US); LOKHOV, Sergey, Sunnyvale California 94089 (US)
(74) Representative: Forresters IP LLP
(86) International application number: PCT/US2013/061743
(87) International publication number: WO 2014/052487

(56) References cited:
- WO-A2-2008/029295
- WO-A2-2010/085966
- WO-A2-2011/103502
- US-A- 6 090 552
- US-A- 6 090 552
- US-A1- 2004 259 116
- US-A1- 2005 147 975
- US-A1- 2006 188 893

## Description

### FIELD OF THE INVENTION

The present invention relates generally to the area of detection and quantification of RNA molecules. In particular, the invention relates to methods and kits for detection and quantification of microRNAs.

### BACKGROUND OF THE INVENTION

MicroRNAs are an abundant class of approximately 22-nucleotide-noncoding RNAs, which play important regulatory roles in animal, plant and virus development. The awareness of microRNAs was initiated almost 15 years ago by the discovery of lin-4, which encode a small RNA involved in the timing and progression of the nematode in C. elegans life cycle and larval development (Lee et al. 1993 Cell 75:843-854, Wightman et al. 1993 Cell 75:855362), but it was only recently recognized that microRNAs form a major class of ribo-regulators that have broad regulatory functions in animals (Lagos-Quintana et al. 2001 Science 294:853858, Lau et al. 2001 Science 294:858-862, Lee and Ambros. 2001 Science 294:862-864).

MicroRNAs are involved in the regulation of gene expression at the posttranscriptional level by degrading or blocking translation of messenger RNA targets, and it has been speculated that approximately 30 % of the human genome could be regulated by microRNAs. microRNAs play roles in various cellular processes including development, growth and proliferation, apoptosis, differentiation, and various human diseases (http://www.mir2disease.org/) such as cancer and diabetes. The importance of microRNAs in cancer is highlighted in a recent article (Barbarotto et al 2008 Int. J. Cancer. 122:969-977), which indicates that: i) miRNAs are altered in every type of analyzed human cancer; (ii) miRNAs act as oncogenes and tumor suppressors; (iii) miRNA alterations may cause cancer predisposition; (iv) miRNA profiling represents a new cancer diagnostic tool; and (v) miRNA profiling represents a prognostic tool in cancer.

Quantification of microRNAs by qRT-PCR procedures is very challenging due to the small size of microRNAs (i.e., only about 21 to 25 nucleotides, which is the size of primers normally used for PCR). Solutions to this problem have been published in Raymond et al. RNA. 2005 Nov;ll(ll): 1737-44; Gilad et al. PLoS ONE. 2008 Sep 5;3(9):e3148; Sharbati-Tehrani et al. BMC Molecular Biology. 2008, 9:34. Raymond et al. describes a qRT-PCR assay that involves a gene-specific reverse transcription step followed by a SYBR green qPCR step using a gene-specific forward primer containing locked nucleic acid (LNA) molecules and a universal reverse primer. Gilad et al. reports a qRT-PCR assay that involves a poladenylation step, a non-specific reverse transcription step, and a qPCR step involving a gene-specific forward primer, a gene-specific TaqMan primer and a universal reverse primer. Sharbati-Tehrani et al. developed a qRT-PCR assay that involves a gene-specific reverse transcription step followed by a SYBR green qPCR step using a gene-specific forward primer and two universal primers.

Diagnostic microRNA assays have been developed by Applied Biosystems, Inc. and Exiqon. The ABI Taqman microRNA assay design is potentially able to multiplex but is complex and requires reverse transcription at low, inefficient temperatures. The technology uses a stem-loop reverse transcription (RT) primer and individual microRNA probe and primer sets. There are a total of four oligonucleotides per target: looped RT primer, forward PCR primer, reverse PCR primer, and TAQMAN^{®} probe. The Exiqon microRNA assay utilizes a universal tag and first-strand synthesis and SYBR^{®} Green for detection. This system is not able to multiplex and utilizes at least three oligonucleotides (degenerate RT primer with universal tag, forward, and reverse primer). WO2008/029295 A2 discloses methods and compositions for detecting, amplifying and labeling targeted nucleic acids. WO2011/130502 A2 discloses materials and methods for detecting, quantifying and/or profiling microRNAs. US 6,090,552 A discloses labeled nucleic acid amplification oligonucleotides. US 20040259116 discloses methods for amplifying nucleic acids.

### SUMMARY OF THE INVENTION

The invention is defined in the claims. One aspect of the disclosure is a method for amplifying a specific RNA molecule in a sample. In some embodiments, the method entails:
(a) adding a poly(ribonucleotide) sequence to RNA molecules in the sample;
(b) reverse transcribing the poly(ribonucleotide)-RNA molecules using a reverse primer including a sequence that anneals to said poly(ribonucleotide) sequence; and
(c) amplifying and detecting the cDNA molecule(s) using the same reverse primer and using a forward primer specific for the RNA molecule to be detected. At least one of the forward and reverse primers includes a hairpin primer including:
   (i) a first nucleotide sequence specific for the RNA molecule to be detected and/or a sequence that anneals to said poly(ribonucleotide) sequence;
   (ii) a second nucleotide sequence at the 5' end of the first sequence;
   (iii) a third nucleotide sequence or spacer at the 5' end of the second sequence;
   (iv) a fourth nucleotide sequence at the 5' end of the third sequence, the fourth sequence being complementary to the second sequence, so as to form a double-stranded duplex; and
   (v) means for emitting a detectable signal when the strands of the duplex are separated; wherein, when the reverse primer includes a hairpin primer, the sequence that anneals to said poly(ribonucleotide) sequence is present in said first nucleotide sequence of the hairpin primer. In some embodiments, the addition of a poly(ribonucleotide) sequence includes poly-adenylation, and the sequence that anneals to said poly(ribonucleotide) sequence includes an oligo(dT) sequence. In some embodiments, the addition of a poly(ribonucleotide) sequence includes poly-uridinylation, and the sequence that anneals to said poly(ribonucleotide) sequence includes an oligo(dA) sequence. In some embodiments, the reverse primer additionally includes a nucleotide sequence specific for the RNA molecule to be detected at the 3' end of the sequence that anneals to said poly(ribonucleotide) sequence. In some embodiments, the forward primer includes a hairpin primer and the reverse primer does not include a hairpin primer. In some embodiments, the reverse primer includes a hairpin primer and the forward primer does not include a hairpin primer.

In some embodiments, the method of any preceding claim, DNA molecules corresponding to at least two specific RNA molecules are amplified and detected in a multiplex amplification reaction using two different primer pairs, each of which is specific for one RNA molecule to be detected. In some embodiments, at least one primer pair includes a hairpin primer. In some embodiments, cDNA molecules corresponding to 3, 4, 5, 6, 7, 8, 9, or 10 specific RNA molecules are amplified and detected in a single multiplex reaction. In some multiplex embodiments, each primer pair employed in the method includes a hairpin primer and is specific for each RNA molecule to be detected.

In some embodiments, the method entails amplifying and detecting one or more mRNA molecule(s) with the non-coding RNA in a multiplex reaction. In some embodiments, the one or more mRNA molecule(s) is/are amplified and detected using a primer pair that includes a hairpin primer. In some embodiments, the one or more mRNA molecule(s) is/are amplified using a primer pair that does not include a hairpin primer. In some embodiments, the one or more mRNA molecule(s) is/are detected using a probe. In some embodiments, the probe(s) is/are specific for the mRNA molecules.

In some embodiments, the addition of a poly(ribonucleotide) sequence and reverse transcribing is carried out in a single reaction mixture. In some embodiments, the amplifying and detecting is carried out in a reaction mixture separate from the addition of a poly(ribonucleotide) sequence and reverse transcribing. In some embodiments, the addition of a poly(ribonucleotide) sequence, reverse transcribing, and amplifying and detecting are all carried out in a single reaction mixture. In some embodiments, the single reaction mixture includes a poly(A)polymerase and a single enzyme for reverse transcribing and amplifying. In some embodiments, the single reaction mixture includes a single enzyme for reverse transcribing and amplifying and an enzyme that adds a poly(U) sequence to RNA molecules, wherein the enzyme is selected from a uridylyl transferase or a poly(U)polymerase. In some embodiments, the amplifying and detecting includes quantitative real-time PCR.

Other aspects of the invention include kits. In some aspects of the disclosure, the kit includes:
(a) a forward primer and a reverse primer, wherein the forward primer is specific for the RNA molecule to be detected, and the reverse primer includes a sequence that anneals to a poly(ribonucleotide) sequence; wherein at least one of the forward and reverse primers includes a hairpin primer including:
   (i) a first nucleotide sequence specific for the RNA molecule to be detected and/or a sequence that anneals to said poly(ribonucleotide) sequence;
   (ii) a second nucleotide sequence at the 5' end of the first sequence;
   (iii) a third nucleotide sequence or spacer at the 5' end of the second sequence;
   (iv) a fourth nucleotide sequence at the 5' end of the third sequence, the fourth sequence being complementary to the second sequence, so as to form a double-stranded duplex; and
   (v) means for emitting a detectable signal when the strands of the duplex are separated; wherein, when the reverse primer includes a hairpin primer, said sequence that anneals to a poly(ribonucleotide) sequence is present in said first nucleotide sequence of the hairpin primer; and
(b) an enzyme capable of adding said poly(ribonucleotide) sequence to RNA molecules.

In some aspects of the disclosure, the kit includes:
a forward primer and a reverse primer, wherein the forward primer is specific for the RNA molecule to be detected, and the reverse primer includes a sequence that anneals to a poly(ribonucleotide) sequence; wherein at least one of the forward and reverse primers includes a hairpin primer including:
(i) a first nucleotide sequence specific for the RNA molecule to be detected and/or a sequence that anneals to said poly(ribonucleotide) sequence;
(ii) a second nucleotide sequence at the 5' end of the first sequence;
(iii) a third nucleotide sequence or spacer at the 5' end of the second sequence;
(iv) a fourth nucleotide sequence at the 5' end of the third sequence, the fourth sequence being complementary to the second sequence, so as to form a double-stranded duplex; and
(v) means for emitting a detectable signal when the strands of the duplex are separated; wherein, when the reverse primer includes a hairpin primer, said sequence that anneals to a poly(ribonucleotide) sequence is present in said first nucleotide sequence of the hairpin primer; and wherein the kit includes no additional primers or probes for amplifying and detecting cDNA molecules corresponding to said one or more specific RNA molecule(s). In some embodiments, this kit can additionally include an enzyme capable of adding said poly(ribonucleotide) sequence to RNA molecules.

In some embodiments of kits, the reverse primer additionally includes a nucleotide sequence specific for the RNA molecule to be detected at the 3' end of said poly(ribonucleotide) sequence. In some embodiments of the kits, the sequence that anneals to said poly(ribonucleotide) sequence includes an oligo(dT) sequence. In some embodiments of the kits, the sequence that anneals to said poly(ribonucleotide) sequence includes an oligo(dA) sequence. In some embodiments of the kits, the forward primer includes a hairpin primer and the reverse primer does not include a hairpin primer. In some embodiments of the kits, the reverse primer includes a hairpin primer and the forward primer does not include a hairpin primer.

In some embodiments of the kits, the enzyme capable of adding said poly(ribonucleotide) sequence to RNA molecules includes a poly(A)polymerase. In some embodiments of the kits, the enzyme capable of adding said poly(ribonucleotide) sequence to RNA molecules includes an enzyme selected from the group consisting of a uridylyl transferase or a poly(U)polymerase. In some embodiments, the kit includes a reverse transcriptase. In some embodiments, the enzyme capable of adding said poly(ribonucleotide) sequence to RNA molecules and the reverse transcriptase are provided in one container. In some embodiments, the kit includes a DNA polymerase. In some embodiments, the kit includes a reverse transcriptase and a DNA polymerase in the form of a single enzyme. In some embodiments, the kit includes an enzyme capable of adding said poly(ribonucleotide) sequence to RNA molecules together with a reverse transcriptase and a DNA polymerase in the form of a single enzyme. In some embodiments, the enzyme capable of adding said poly(ribonucleotide) sequence to RNA molecules together with the reverse transcriptase and DNA polymerase in the form of a single enzyme are provided in one container.

In some embodiments, the kit includes at least two different primer pairs, each of which is specific for one RNA molecule to be detected. In some embodiments, at least one primer pair includes a hairpin primer. In some embodiments, the kit includes at least 3, 4, 5, 6, 7, 8, 9, or 10 different primer pairs, each of which is specific for one RNA molecule to be detected. In some embodiments, each hairpin primer in the kit produces a different detectable signal.

In some embodiments, the kit additionally includes one or more primer pair(s) for amplifying one or more mRNA molecule(s) with the non-coding RNA in a multiplex reaction. In some embodiments, the one or more primer pair(s) for amplifying one or more mRNA molecule(s) do/does not include a hairpin primer. In some embodiments, the kit additionally includes one or more probe(s) for detecting the mRNA molecules. In some embodiments, the probe(s) is/are specific for the mRNA molecules.

In some embodiments of the methods and kits, the specific RNA molecule includes a non-coding RNA. In some embodiments, the non-coding RNA is selected from: small nuclear RNAs (snRNAs), small nucleolar RNA (snoRNA), small temporal RNAs (stRNAs), antigene RNAs (ag RNAs), microRNA (miRNA), small interfering RNA (siRNA), and Piwi-interacting RNAs (piRNA). In some embodiments, the non-coding RNA is selected from one or more of: miR-31, CTL23, miR-126, miR-1, miR-335, miR-139-5p, miR-210, miR-143, miR-10b, miR-181a, miR-28-5p, miR-127-5p, miR-125b, miR-222, miR-29a*, miR-381, let7d, miR-93, miR-200-5p, miR-221, and miR-107.

In some embodiments of the methods and kits, the signal-emitting means includes an energy donor moiety and an energy acceptor moiety, each bound to the hairpin primer and spaced such that the signal is detectable only when the strands of the duplex are separated. In some embodiments, the energy donor and acceptor moieties are spaced a distance in the range of about 10-40 nucleotides. In some embodiments, the acceptor moiety is a fluorophore that emits fluorescent light at a wavelength different than that emitted by the donor moiety. In some embodiments, the energy donor moiety is a fluorophore and the energy acceptor moiety is a fluorophore quencher. In some embodiments, the donor moiety is selected from: fluorescein, 5-carboxyfluorescein (FAM), rhodamine, 5-(2'-aminoethyl) aminonapthalene-1-sulfonic acid (EDANS), anthranilamide, coumarin, terbium chelate derivatives, and Reactive Red 4, and the acceptor moiety is selected from: DABCYL, rhodamine, tetramethyl rhodamine, pyrene butyrate, eosine nitrotyrosine, ethidium, fluorescein, Malachite green, and Texas Red. In some embodiments, the donor moiety is fluorescein or a derivative thereof, and the acceptor moiety is DABCYL. In some embodiments, the energy donor moiety and the energy acceptor moiety are situated on opposite strand nucleotides that are five, or fewer, nucleotides apart in the duplex. In some embodiments, the energy donor moiety and the energy acceptor moiety are situated on complementary nucleotides that are opposite each other in the duplex.

### BRIEF DESCRIPTION OF THE DRAWINGS

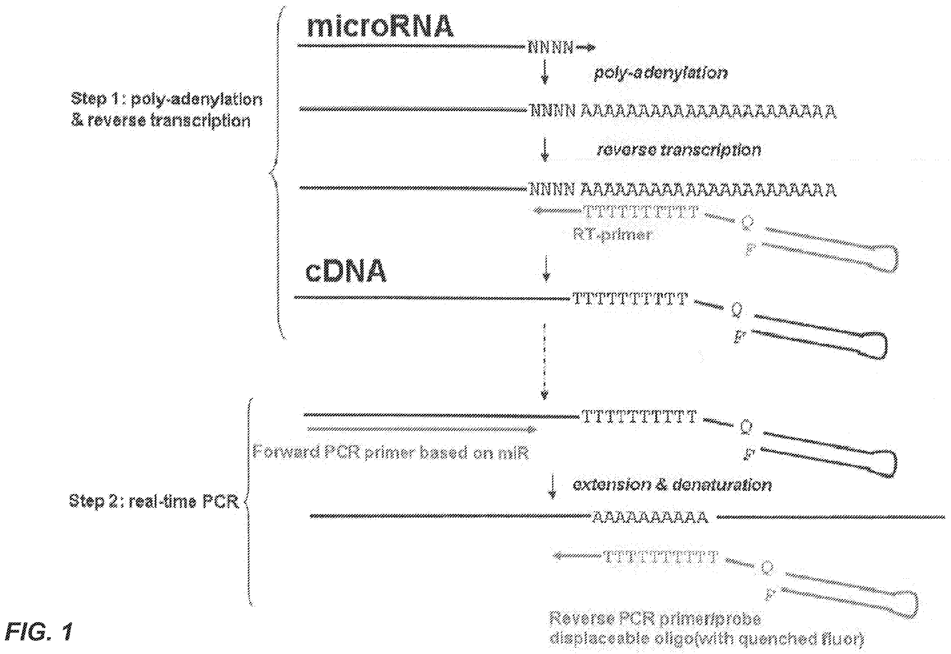

Figure 1 illustrates an embodiment of the method described herein in which a reverse primer is a hairpin primer.

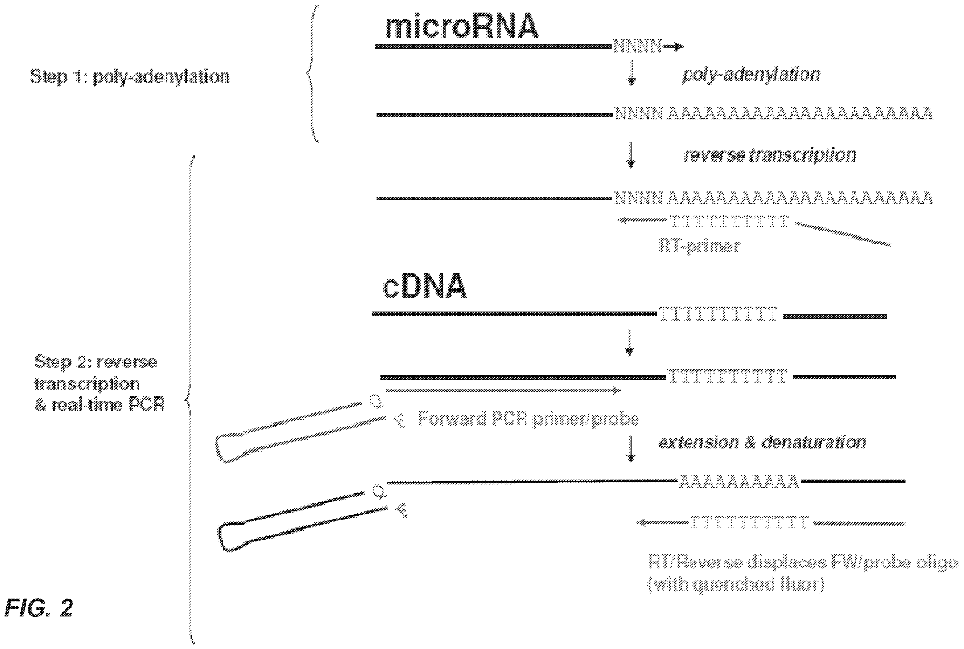

Figure 2 illustrates an embodiment of the method described herein in which a forward primer is a hairpin primer.
Figure 3 shows the results of quantitative real-time PCR microRNA assays carried according to the methods described herein in singleplex with a dilution series of target. Fluorescence is plotted against PCR cycle number.
Figure 4A-B shows the results of a multiplex quantitative real-time PCR microRNA assay. Fluorescence is plotted against PCR cycle number. (A) miR31; (B) CTL23.

### DETAILED DESCRIPTION

The invention is defined in the claims. The present invention relates to measurement of RNA (e.g., miRNA) markers of a genetic state with two oligonucleotides (combination RT/reverse primer/probe and forward primer or RT/reverse primer and forward primer/probe) for each target which can be made specific for multiple targets in multiplex microRNA assays.

### Definitions

Terms used in the claims and specification are defined as set forth below unless otherwise specified.

The term "nucleic acid" refers to a nucleotide polymer, and unless otherwise limited, includes known analogs of natural nucleotides that can function in a similar manner (e.g., hybridize) to naturally occurring nucleotides.

The term nucleic acid includes any form of DNA or RNA, including, for example, genomic DNA; complementary DNA (cDNA), which is a DNA representation of mRNA, usually obtained by reverse transcription of messenger RNA (mRNA) or by amplification; DNA molecules produced synthetically or by amplification; mRNA; and non-coding RNA.

The term nucleic acid encompasses double- or triple-stranded nucleic acid complexes, as well as single-stranded molecules. In double- or triple-stranded nucleic acid complexes, the nucleic acid strands need not be coextensive (i.e, a double-stranded nucleic acid need not be double-stranded along the entire length of both strands).

The term nucleic acid also encompasses any chemical modifications thereof, such as by methylation and/or by capping. Nucleic acid modifications can include addition of chemical groups that incorporate additional charge, polarizability, hydrogen bonding, electrostatic interaction, and functionality to the individual nucleic acid bases or to the nucleic acid as a whole. Such modifications may include base modifications such as 2'-position sugar modifications, 5-position pyrimidine modifications, 8-position purine modifications, modifications at cytosine exocyclic amines, substitutions of 5-bromo-uracil, sugar-phosphate backbone modifications, unusual base pairing combinations such as the isobases isocytidine and isoguanidine, and the like.

More particularly, in some embodiments, nucleic acids, can include polydeoxyribonucleotides (containing 2-deoxy-D-ribose), polyribonucleotides (containing D-ribose), and any other type of nucleic acid that is an N- or C-glycoside of a purine or pyrimidine base, as well as other polymers containing nonnucleotidic backbones, for example, polyamide (e.g., peptide nucleic acids (PNAs)) and polymorpholino polymers (commercially available from the Anti-Virals, Inc., Corvallis, Oregon, as Neugene), and other synthetic sequence-specific nucleic acid polymers providing that the polymers contain nucleobases in a configuration which allows for base pairing and base stacking, such as is found in DNA and RNA. The term nucleic acid also encompasses locked nucleic acids (LNAs), which are described in U.S. Patent Nos. 6,794,499, 6,670,461, 6,262,490, and 6,770,748.

The nucleic acid(s) can be derived from a completely chemical synthesis process, such as a solid phase-mediated chemical synthesis, from a biological source, such as through isolation from any species that produces nucleic acid, or from processes that involve the manipulation of nucleic acids by molecular biology tools, such as DNA replication, PCR amplification, reverse transcription, or from a combination of those processes.

Non-coding RNAs include those RNA species that are not necessarily translated into protein. These include, but are not limited to, transfer RNA (tRNA) and ribosomal RNA (rRNA), as well as RNAs such as small nuclear RNAs (snRNAs), small nucleolar RNAs (snoRNAs; e.g., those associated with methylation or pseudouridylation), small temporal RNAs (stRNAs), antigene RNAs (ag RNAs), microRNAs (miRNAs; which regulate gene expression), small interfering RNAs (siRNAs; which are involved in the RNA interference (RNAi) pathway, where they interfere with the expression of specific genes, but have also been shown to act as antiviral agents and in shaping the chromatin structure of a genome), and Piwi-interacting RNAs (piRNAs; which form RNA-protein complexes through interactions with Piwi proteins; these piRNA complexes have been linked to transcriptional gene silencing of retrotransposons and other genetic elements in germ line cells, particularly those in spermatogenesis), and long non-coding RNAs (long ncRNAs; which are non-coding transcripts that are typically longer than about 200 nucleotides).

The terms "microRNA," "miRNA," and "miR" are used synonymously and refer to non-coding RNAs derived from endogenous genes of living organisms such as animals and plants. In "mature" form, these miRNAs are typically 21-25 nucleotides in length and ar biologically active. They are generally processed from longer hairpin-like precursors termed pre-miRNAs (pre-miRs) having a length of approximately 75 nucleotides. The precursors of pre-miRNAs are pri-miRNAs (pri-miRs), with a length of about 500 to 3000 nucleotides. MiRNAs assemble in complexes termed miRNPs and act as major regulators on important biological processes by binding to messenger RNA and interfering with translation efficiency. Target microRNAs of the methods described herein refer to all known microRNAs, such as microRNAs known from the scientific literature and public databases, such as the miRBase database (microrna.sanger.ac.uk/), which is the home of microRNA data on the web administrated by the Sanger Institute, UK.

"Polyadenylating" refers to the addition of a poly(A) tail, a stretch of RNA where all the bases are adenines, at the 3'-terminus of an RNA molecule. Polyadenylation is a natural biological process in living organisms, but it can also be carried out in vitro using various polymerases such as commercial available *E. coli* Poly(A) Polymerase I (E-PAP).

"Polyuridinylating" refers to the addition of a poly(U) tail, a stretch of RNA where all the bases are uridines, at the 3'-terminus of an RNA molecule. Polyuridinylating is a natural biological process in living organisms, but it can also be carried out in vitro using various enzymes, such as uridylyl transferase and poly(U)polymerase, the latter of which is commercially available from New England Biolabs.

As used herein, the term "complementary" refers to the capacity for precise pairing between two nucleotides; i.e., if a nucleotide at a given position of a nucleic acid is capable of hydrogen bonding with a nucleotide of another nucleic acid to form a canonical base pair, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. The degree of complementarity between nucleic acid strands has significant effects on the efficiency and strength of hybridization between nucleic acid strands.

"Specific hybridization" refers to the binding of a nucleic acid to a target nucleotide sequence in the absence of substantial binding to other nucleotide sequences present in the hybridization mixture under defined stringency conditions. Those of skill in the art recognize that relaxing the stringency of the hybridization conditions allows sequence mismatches to be tolerated.

In some embodiments, hybridizations are carried out under stringent hybridization conditions. The phrase "stringent hybridization conditions" generally refers to a temperature in a range from about 5°C to about 20°C or 25°C below than the melting temperature (Tₘ) for a specific sequence at a defined ionic strength and pH. As used herein, the Tₘ is the temperature at which a population of double-stranded nucleic acid molecules becomes half-dissociated into single strands. Methods for calculating the Tₘ of nucleic acids are well known in the art (see, e.g., Berger and Kimmel (1987) METHODS IN ENZYMOLOGY, VOL. 152: GUIDE TO MOLECULAR CLONING TECHNIQUES, San Diego: Academic Press, Inc. and Sambrook et al. (1989) MOLECULAR CLONING: A LABORATORY MANUAL, 2ND ED., VOLS. 1-3, Cold Spring Harbor Laboratory). As indicated by standard references, a simple estimate of the Tₘ value may be calculated by the equation: Tₘ =81.5+0.41(% G+C), when a nucleic acid is in aqueous solution at 1 M NaCl (see, e.g., Anderson and Young, Quantitative Filter Hybridization in NUCLEIC ACID HYBRIDIZATION (1985)). The melting temperature of a hybrid (and thus the conditions for stringent hybridization) is affected by various factors such as the length and nature (DNA, RNA, base composition) of the primer or probe and nature of the target nucleic acid (DNA, RNA, base composition, present in solution or immobilized, and the like), as well as the concentration of salts and other components (e.g., the presence or absence of formamide, dextran sulfate, polyethylene glycol). The effects of these factors are well known and are discussed in standard references in the art. Illustrative stringent conditions suitable for achieving specific hybridization of most sequences are: a temperature of at least about 60°C and a salt concentration of about 0.2 molar at pH7. Tm calculation for oligonuclotide sequences based on nearest-neighbors thermodynamics can carried out as described in "A unified view of polymer, dumbbell, and oligonucleotide DNA nearest-neighbor thermodynamics" John SantaLucia, Jr., PNAS February 17, 1998 vol. 95 no. 4 1460-1465.

The term "oligonucleotide" is used to refer to a nucleic acid that is relatively short, generally shorter than 200 nucleotides, more particularly, shorter than 100 nucleotides, most particularly, shorter than 50 nucleotides. Typically, oligonucleotides are single-stranded DNA molecules.

The term "primer" refers to an oligonucleotide that is capable of hybridizing (also termed "annealing") with a nucleic acid and serving as an initiation site for nucleotide (RNA or DNA) polymerization under appropriate conditions (i.e., in the presence of four different nucleoside triphosphates and an agent for polymerization, such as DNA or RNA polymerase or reverse transcriptase) in an appropriate buffer and at a suitable temperature. The appropriate length of a primer depends on the intended use of the primer, but primers are typically at least 7 nucleotides long and, in some embodiments, range from 10 to 30 nucleotides, or, in some embodiments, from 15 to 30 nucleotides, in length. In some embodiments, primers can be somewhat longer, e.g., 30 to 50 nucleotides long. In this context, "primer length" refers to the portion of an oligonucleotide or nucleic acid that hybridizes to a complementary "target" sequence and primes nucleotide synthesis. Short primer molecules generally require cooler temperatures to form sufficiently stable hybrid complexes with the template. A primer need not reflect the exact sequence of the template but must be sufficiently complementary to hybridize with a template.

A primer is said to anneal to another nucleic acid if the primer, or a portion thereof, hybridizes to a nucleotide sequence within the nucleic acid. The statement that a primer hybridizes to a particular nucleotide sequence is not intended to imply that the primer hybridizes either completely or exclusively to that nucleotide sequence. For example, in some embodiments, amplification primers used herein are said to "anneal to" or be "specific for" a nucleotide sequence." This description encompasses primers that anneal wholly to the nucleotide sequence, as well as primers that anneal partially to the nucleotide sequence.

The term "primer pair" refers to a set of primers including a 5' "upstream primer" or "forward primer" that hybridizes with the complement of the 5' end of the DNA sequence to be amplified and a 3' "downstream primer" or "reverse primer" that hybridizes with the 3' end of the sequence to be amplified. As will be recognized by those of skill in the art, the terms "upstream" and "downstream" or "forward" and "reverse" are not intended to be limiting, but rather provide illustrative orientation in some embodiments.

A "probe" is a nucleic acid capable of binding to a target nucleic acid of complementary sequence through one or more types of chemical bonds, generally through complementary base pairing, usually through hydrogen bond formation, thus forming a duplex structure. The probe can be labeled with a detectable label to permit facile detection of the probe, particularly once the probe has hybridized to its complementary target. Alternatively, however, the probe may be unlabeled, but may be detectable by specific binding with a ligand that is labeled, either directly or indirectly. Probes can vary significantly in size. Generally, probes are at least 7 to 15 nucleotides in length. Other probes are at least 20, 30, or 40 nucleotides long. Still other probes are somewhat longer, being at least 50, 60, 70, 80, or 90 nucleotides long. Yet other probes are longer still, and are at least 100, 150, 200 or more nucleotides long. Probes can also be of any length that is within any range bounded by any of the above values (e.g., 15-20 nucleotides in length).

The primer or probe can be perfectly complementary to the target nucleic acid sequence or can be less than perfectly complementary. In some embodiments, the primer has at least 65% identity to the complement of the target nucleic acid sequence over a sequence of at least 7 nucleotides, more typically over a sequence in the range of 10-30 nucleotides, and, in some embodiments, over a sequence of at least 14-25 nucleotides, and, in some embodiments, has at least 75% identity, at least 85% identity, at least 90% identity, or at least 95%, 96%, 97%. 98%, or 99% identity. It will be understood that certain bases (e.g., the 3' base of a primer) are generally desirably perfectly complementary to corresponding bases of the target nucleic acid sequence. Primer and probes typically anneal to the target sequence under stringent hybridization conditions.

As used herein with reference to a portion of a primer or a nucleotide sequence within the primer, the term "specific for" a nucleic acid, such as "the RNA molecule to be detected" refers to a primer or nucleotide sequence that can specifically anneal to the target nucleic acid under suitable annealing conditions.

Amplification according to the present teachings encompasses any means by which at least a part of at least one target nucleic acid is reproduced, typically in a template-dependent manner, including without limitation, a broad range of techniques for amplifying nucleic acid sequences, either linearly or exponentially. Illustrative means for performing an amplifying step include ligase chain reaction (LCR), ligase detection reaction (LDR), ligation followed by Q-replicase amplification, PCR, primer extension, strand displacement amplification (SDA), hyperbranched strand displacement amplification, multiple displacement amplification (MDA), nucleic acid strand-based amplification (NASBA), twostep multiplexed amplifications, rolling circle amplification (RCA), and the like, including multiplex versions and combinations thereof, for example but not limited to, OLA/PCR, PCR/OLA, LDR/PCR, PCR/PCR/LDR, PCR/LDR, LCR/PCR, PCR/LCR (also known as combined chain reaction--CCR), and the like. Descriptions of such techniques can be found in, among other sources, Ausbel et al.; PCR Primer: A Laboratory Manual, Diffenbach, Ed., Cold Spring Harbor Press (1995); The Electronic Protocol Book, Chang Bioscience (2002); Msuih et al., J. Clin. Micro. 34:501-07 (1996); The Nucleic Acid Protocols Handbook, R. Rapley, ed., Humana Press, Totowa, N.J. (2002); Abramson et al., Curr Opin Biotechnol. 1993 Feb.;4(1):41-7, U.S. Pat. No. 6,027,998; U.S. Pat. No. 6,605,451, Barany et al., PCT Publication No. WO 97/31256; Wenz et al., PCT Publication No. WO 01/92579; Day et al., Genomics, 29(1): 152-162 (1995), Ehrlich et al., Science 252: 1643-50 (1991); Innis et al., PCR Protocols: A Guide to Methods and Applications, Academic Press (1990); Favis et al., Nature Biotechnology 18:561-64 (2000); and Rabenau et al., Infection 28:97-102 (2000); Belgrader, Barany, and Lubin, Development of a Multiplex Ligation Detection Reaction DNA Typing Assay, Sixth International Symposium on Human Identification, 1995 (available on the world wide web at:
promega.com/geneticidproc/ussymp6proc/blegrad.html- ); LCR Kit Instruction Manual, Cat. #200520, Rev. #050002, Stratagene, 2002; Barany, Proc. Natl. Acad. Sci. USA 88:188-93 (1991); Bi and Sambrook, Nucl. Acids Res. 25:2924-2951 (1997); Zirvi et al., Nucl. Acid Res. 27:e40i-viii (1999); Dean et al., Proc Natl Acad Sci USA 99:5261-66 (2002); Barany and Gelfand, Gene 109:1-11 (1991); Walker et al., Nucl. Acid Res. 20:1691-96 (1992); Polstra et al., BMC Inf. Dis. 2:18- (2002); Lage et al., Genome Res. 2003 Feb.;13(2):294-307, and Landegren et al., Science 241:1077-80 (1988), Demidov, V., Expert Rev Mol Diagn. 2002 Nov.;2(6):542-8., Cook et al., J Microbiol Methods. 2003 May;53(2):165-74, Schweitzer et al., Curr Opin Biotechnol. 2001 Feb.;12(1):21-7, U.S. Pat. No. 5,830,711, U.S. Pat. No. 6,027,889, U.S. Pat. No. 5,686,243, PCT Publication No. WO0056927A3, and PCT Publication No. WO9803673A1.

In some embodiments, amplification comprises at least one cycle of the sequential procedures of: annealing at least one primer with complementary or substantially complementary sequences in at least one target nucleic acid; synthesizing at least one strand of nucleotides in a template-dependent manner using a polymerase; and denaturing the newly-formed nucleic acid duplex to separate the strands. The cycle may or may not be repeated. Amplification can comprise thermocycling or can be performed isothermally.

A "multiplex amplification reaction" is one in which two nucleic acids are amplified simultaneously and detected by means of distinguishable labels.

The term "qPCR" is used herein to refer to quantitative real-time polymerase chain reaction (PCR), which is also known as "real-time PCR" or "kinetic polymerase chain reaction;" all terms refer to PCR with real-time signal detection.

A "reagent" refers broadly to any agent used in a reaction, other than the analyte (e.g., nucleic acid being analyzed). Illustrative reagents for a nucleic acid amplification reaction include, but are not limited to, buffer, metal ions, polymerase, reverse transcriptase, primers, template nucleic acid, nucleotides, labels, dyes, nucleases, dNTPs, and the like. Reagents for enzyme reactions include, for example, substrates, cofactors, buffer, metal ions, inhibitors, and activators.

The term "label," as used herein, refers to any atom or molecule that can be used to provide a detectable and/or quantifiable signal. In particular, the label can be attached, directly or indirectly, to a nucleic acid or protein. Suitable labels that can be attached to probes include, but are not limited to, radioisotopes, fluorophores, chromophores, mass labels, electron dense particles, magnetic particles, spin labels, molecules that emit chemiluminescence, electrochemically active molecules, enzymes, cofactors, and enzyme substrates.

The term "dye," as used herein, generally refers to any organic or inorganic molecule that absorbs electromagnetic radiation at a wavelength greater than or equal 300 nm.

The term "fluorescent dye," as used herein, generally refers to any dye that emits electromagnetic radiation of longer wavelength by a fluorescent mechanism upon irradiation by a source of electromagnetic radiation, such as a lamp, a photodiode, or a laser or another fluorescent dye.

### Methods for Amplifying Specific RNA Molecules

One aspect of the invention includes a method for amplifying a specific RNA molecule in a sample, typically an RNA molecule that does not have a poly(A) tail. The method entails adding a poly(ribonucleotide) sequence to RNA molecules in the sample and reverse transcribing the resultant RNA molecules using a reverse primer including sequence that anneals to the poly(ribonucleotide) sequence to produce at least one cDNA molecule if the target RNA molecule is present in the sample. The cDNA molecule(s) are amplified and detected using the same reverse primer as was used to generate the cDNA molecule(s) and a forward primer specific for the RNA molecule to be detected. At least one of the forward and reverse primers includes a hairpin primer with:
(i) a first nucleotide sequence specific for the RNA molecule to be detected and/or a sequence that anneals to the poly(ribonucleotide) sequence;
(ii) a second nucleotide sequence at the 5' end of the first sequence;
(iii) a third nucleotide sequence or spacer at the 5' end of the second sequence;
(iv) a fourth nucleotide sequence at the 5' end of the third sequence, the fourth sequence being complementary to the second sequence, so as to form a double-stranded duplex; and
(v) means for emitting a detectable signal when the strands of the duplex are separated;
wherein, when the reverse primer is a hairpin primer, the sequence that anneals to the poly(ribonucleotide) sequence is present in the first nucleotide sequence of the hairpin primer. In some embodiments, the reverse primer additionally includes a nucleotide sequence specific for the RNA molecule to be detected at the 3' end of the sequence that anneals to the poly(ribonucleotide) sequence.

In some embodiments, the method is carried out in a multiplex format in which cDNA molecules corresponding to at least two specific RNA molecules are amplified and detected in a multiplex amplification reaction using two different primer pairs, each of which is specific for one RNA molecule to be detected. In some embodiments, at least one primer pair includes a hairpin primer, more than one primer pair includes a hairpin primer, or all primer pairs in the reaction include a hairpin primer. Each hairpin primer in a single multiplex reaction generally includes a means for emitting a detectable signal when the strands of the duplex are separated that is distinguishable from all others in the reaction. The number of cDNAs that can be amplified and detected in a single multiplex reaction depends, partly, on the number of signals that can be distinguished from one another in one reaction. In some embodiments, cDNA molecules corresponding to 3, 4, 5, 6, 7, 8, 9, or 10 specific RNA molecules are amplified and detected in a single multiplex reaction.

In some embodiments, the method includes amplifying and detecting one or more mRNA molecule(s) with the non-coding RNA in a multiplex reaction. The mRNA molecule(s) can, but need not, be amplified and detected using a primer pair that includes a hairpin primer. For example, the mRNA molecule(s) can be amplified by unlabeled primers and detected using a probe that is labeled or that becomes labeled during the assay. In some embodiments, the probe(s) is/are specific for the mRNA molecules. In some embodiments, mRNA molecules can be amplified using unlabeled primers and detected using a DNA-binding dye. Suitable primers for amplifying mRNA molecules includes target-specific primers, primer pairs in which one primer includes a oligo(dT) sequence or degenerate sequence.

In some embodiments, the poly(ribonucleotide) sequence includes a poly(A) sequence, and the sequence that anneals to the poly(A) sequence includes an oligo(dT) sequence. In some embodiments, poly(A)polymerase is employed to poly-adenylate RNA molecules. Methods for poly-adenylating and reverse-transcribing RNA molecules are well known and described in Sambrook, J., Fritsch, E.F., and Maniatis, T., Molecular Cloning: A Laboratory Manual. Cold Spring Harbor Laboratory Press, NY, Vol. 1, 2, 3 (1989), for example. In some embodiments, the target RNA molecule is poly-adenylated and reverse transcribed in a single reaction mixture. Suitable procedures are described in PCT Publication No. WO 2010/985966, published August 5, 2010. Briefly, poly(A)polymerase, reverse transcriptase, forward and reverse primers, dNTPs, ATP, and an RNA sample are incubated together in a suitable buffer (e.g., a poly(A)polymerase buffer) under suitable conditions to permit poly-adenylation and reverse transcription to produce cDNA molecule(s) corresponding to the target RNA molecule(s). See Example 1.

In some embodiments, the poly(ribonucleotide) sequence includes a poly(U) sequence, and the sequence that anneals to the poly(U) sequence includes an oligo(dA) sequence. Enzymes that are capable of poly-uridinylation include uridylyl transferase or a poly(U)polymerase. These enzymes and their activities are described in Martin & Keller (2007) "RNA-specific ribonucleotidyl transferases," RNA 13:1834-1849. In some embodiments, the poly(ribonucleotide) sequence is added using a ligase.

In some embodiments, amplification and detection of the cDNA molecule(s) can be carried out in a separate reaction mixture, i.e., separate from addition of the poly(ribonucleotide) sequence and reverse transcription. In some embodiments, amplification and detection are carried out by quantitative real-time PCR. Methods for performing real-time PCR are well-known. In some embodiments, real-time PCR reactions include a polymerase (e.g. Taq polymerase), forward and reverse primers, dNTPs, template nucleic acid, and a suitable buffer, which are subjected to thermal cycling to produce amplicons for each primer pair. Real-time PCR can be performed using any PCR instrumentation available in the art. In the methods described herein the same forward and reverse primers are used for amplification/detection as are used for addition of a poly(ribonucleotide) sequence /reverse transcription. Because one of the primers is a hairpin primer that also serves as a probe (see below), only two oligonucleotides (i.e., forward and reverse primers and no separate probe) are needed for target RNA molecule can be detected in a single reaction. Typically, instrumentation used in real-time PCR data collection and analysis includes a thermal cycler, optics for fluorescence excitation and emission collection, and optionally a computer and data acquisition and analysis software.

In some embodiments, addition of a poly(ribonucleotide) sequence, reverse transcription, and amplification/detection are carried out in a single reaction mixture. In some embodiments, a single reaction mixture includes a poly(A) polymerase and a single enzyme for reverse transcribing and amplifying, e.g., a single heat stable DNA polymerase capable of use in reverse transcription-PCR (RT-PCR) procedure. In some embodiments, the single enzyme is HAWKZ05 FAST DNA polymerase, which is commercially available from Roche. In some embodiments, a single reaction mixture includes a single enzyme for reverse transcribing and amplifying and an enzyme that adds a poly(U) sequence to RNA molecules, such as a uridylyl transferase or a poly(U)polymerase.

### Samples

Preparations of nucleic acid-containing samples can be obtained from biological sources and prepared using conventional methods known in the art. In particular, RNA useful in the methods described herein can be obtained from any source, including unicellular organisms and higher organisms such as plants or non-human animals, e.g., canines, felines, equines, primates, and other non-human mammals, as well as humans. Nucleic acids can be obtained from cells, bodily fluids (e.g., blood, a blood fraction, urine, etc.), or tissue samples by any of a variety of standard techniques. In some embodiments, the method employs samples of plasma, serum, spinal fluid, lymph fluid, peritoneal fluid, pleural fluid, oral fluid, and external sections of the skin; samples from the respiratory, intestinal genital, and urinary tracts; samples of tears, saliva, blood cells, stem cells, or tumors. Samples can be obtained from live or dead organisms or from in vitro cultures. Illustrative samples can include single cells, paraffin-embedded tissue samples, and needle biopsies.

Nucleic acids of interest can be isolated using methods well known in the art, with the choice of a specific method depending on the source, the nature of nucleic acid, and similar factors. The sample nucleic acids need not be in pure form, but are typically sufficiently pure to allow the steps of the methods of the invention to be performed.

### Target RNA Molecules

Any target RNA molecule that can be polyadenylated and amplified can be detected using the methods described herein. In some embodiments, at least some nucleotide sequence information will be known for the target RNA molecule(s). For example, sufficient sequence information is generally available for a given target RNA molecule to permit design of at least a suitable forward primer for amplification. In some embodiments, sufficient sequence at the 3' end of the target RNA molecule is known to permit design of a primer that anneals in part to this known sequence and in part to the poly(A) tail produced in the first step of the method. In some embodiments, the targets can include RNAs for which over- or under-expression is indicative of disease, that are expressed in a tissue- or developmental-specific manner, or that are induced by particular stimuli.

In some embodiments, at least one target RNA molecule is a non-coding RNA. In some embodiments, suitable non-coding RNA targets include snRNAs, snoRNAs, stRNAs, agRNAs, miRNAs, siRNAs, piRNAs, and long ncRNAs. In some embodiments, the target RNA molecule incudes any known, or yet to be discovered, microRNAs, such as microRNAs known from the scientific literature and public databases, such as the miRBase database release 19 (microrna.sanger.ac.uk/), including all the mature miRNA and premature miRNA sequences disclosed therein. In some embodiments, microRNA target that can be detected and quantified in the methods described herein include those shown in the following table, which are deregulated in breast cancer.

| **microRNA** | **SEQUENCE** | **DCIS / IDC** | **P-value** - **Wilcoxon test** | SEQ ID NO. |
|---|---|---|---|---|
| miR-126 | TCGTACCGTGAGTAATAATGCG | DOWN | <0.0001 | (SEQ ID NO:1) |
| miR-1 | TGGAATGTAAAGAAGTATGTAT | DOWN | <0.0001 | (SEQ ID NO:2) |
| miR-335 | TCAAGAGCAATAACGAAAAATG | DOWN | <0.0001 | (SEQ ID NO:3) |
| miR-139-5p | TCTACAGTGCACGTGTCTCCAGT | DOWN | <0.0001 | (SEQ ID NO:4) |
| miR-210 | CTGTGCGTGTGACAGCGGCTGA | UP | <0.0001 | (SEQ ID NO:5) |
| miR-143 | TGAGATGAAGCACTGTAGCTC | DOWN | <0.0001 | (SEQ ID NO:6) |
| miR-10b | TACCCTGTAGAACCGAATTTGT | DOWN | <0.0001 | (SEQ ID NO:7) |
| miR-181a | AACATTCAACGCTGTCGGTGAGT | UP | <0.001 | (SEQ ID NO:8) |
| miR-28-5p | AAGGAGCTCACAGTCTATTGA | UP | <0.001 | (SEQ ID NO:9) |
| miR-127-5p | CTGAAGCTCAGAGGGCTCTGAT | DOWN | <0.001 | (SEQ ID NO:10) |
| miR-125b | TCCCTGAGACCCTAACTTGTGA | DOWN | <0.001 | (SEQ ID NO:11) |
| miR-222 | AGCTACATCTGGCTACTGGGT | UP | <0.001 | (SEQ ID NO:12) |
| miR-29a* | ACTGATTTCTTTTGGTGTTCAGA | UP | <0.001 | (SEQ ID NO:13) |
| miR-381 | TATACAAGGGCAAGCTCTCTGT | DOWN | <0.001 | (SEQ ID NO:14) |
| let-7d | AGAGGTAGTAGGTTGCATAGTT | UP | <0.001 | (SEQ ID NO:15) |
| miR-93 | CAAAGTGCTGTTCGTGCAGGTAG | UP | <0.001 | (SEQ ID NO:16) |
| miR-299-5p | TGGTTTACCGTCCCACATACAT | DOWN | <0.001 | (SEQ ID NO:17) |
| miR-221 | AGCTACATTGTCTGCTGGGTTT | UP | <0.001 | (SEQ ID NO:18) |
| miR-107 | AGCAGCATTGTACAGGGCTAT | UP | <0.001 | (SEQ ID NO:19) |

In Example 1, two target miRNA molecules miR31 and CTL23 are detected in a multiplex assay.

### Forward Primers

In some embodiments, the forward primer is not a hairpin primer and has a length in the range of from 10 to 100 nucleotides, such as 10 to 30 or 12 to 25 or 13, 14, 15, 16, 17, 18, 19, 20, 21, 22, 23, or 25 nucleotides. In some embodiments, the length of the forward primer falls within a range bounded by any of these values, e.g., 20 to 23 nucleotides. Considerations for designing non-hairpin forward primers suitable for use in the methods described herein are given in PCT Publication No. WO 2010/085966.

### Reverse Primers

In some embodiments, the reverse primer employed in the method is not a hairpin primer and has a length in the range of from 10 to 100 nucleotides, such as a length in the range from 15 to 50 or 20 to 45 or 21, 22, 23, 24, 25, 26, 27, 28, 29, 30, 31, 32, 33, 34, 35, 36, 37, 38, 39, 40, 41, 42, 43, or 44 nucleotides. In some embodiments, the length of the reverse primer falls within a range bounded by any of these values, e.g., 30 to 45 nucleotides. Considerations for designing non-hairpin reverse primers suitable for use in the methods described herein are given in PCT Publication No. WO 2010/085966.

### Hairpin Primers

In the method described above, at least one of the primers in a primer pair for detecting a specific RNA molecule is a hairpin primer. Hairpin primers utilize the principle of MET between an energy donor moiety and an energy acceptor moiety. In some embodiments, the MET is fluorescence resonance energy transfer (FRET), in which the primers are labeled with donor and acceptor moieties, wherein the donor moiety is a fluorophore and the acceptor moiety may be a fluorophore, such that fluorescent energy emitted by the donor moiety is absorbed by the acceptor moiety. In some embodiments, the acceptor moiety is a fluorophore that releases the energy absorbed from the donor at a different wavelength; the emissions of the acceptor may then be measured to assess the progress of the amplification reaction. In some embodiments, the hairpin primer is labeled with a donor-quencher FRET pair. In some embodiments, the hairpin primer is configured such that the acceptor moiety quenches the fluorescence of the donor. When the primer is incorporated into the amplification product its configuration changes, quenching is eliminated (i.e., primer is linearized), and the fluorescence of the donor moiety may be detected.

Hairpin primers can be any suitable size, and in some embodiments, are in the range of 20 to100 or 30 to 80 or 35 to 60 or 36, 37, 38, 39, 40, 41, 42, 43, 44, 45, 46, 47, 48, 49, 50, 51, 52, 53, 54, 55, 56, 57, 58, or 59 nucleotides. In some embodiments, the length of the hairpin primer falls within a range bounded by any of these values, e.g., 45 to 55 nucleotides. Considerations for designing non-hairpin reverse primers suitable for use in the methods described herein are given in U.S. Patent No. 5,866,336 (issued Feb. 2, 1999).

In some embodiments, the hairpin primer comprises, or alternatively consists of, the following contiguous sequences: (a) at the 3' end of said primer, a first, single-stranded nucleotide sequence of 8-40 nucleotides that comprises at its 3' end a sequence sufficiently complementary to one or more target sequence(s) so as to be able to prime synthesis by a nucleic acid polymerase of a nucleotide sequence complementary to a nucleic acid strand comprising said target sequence; (b) a second nucleotide sequence of 6-30 nucleotides at the 5' end of the first sequence, wherein a nucleotide within said first nucleotide sequence is labeled with a first moiety selected from the group consisting of a donor moiety and an acceptor moiety of a molecular energy transfer pair, wherein the donor moiety emits energy of one or more particular wavelengths when excited, and the acceptor moiety absorbs energy at one or more particular wavelengths emitted by the donor moiety; (c) a third, single-stranded nucleotide sequence of 3-20 nucleotides at the 5' end of said second sequence; (d) a fourth nucleotide sequence of 6-30 nucleotides at the 5' end of the third sequence, wherein a nucleotide within said fourth nucleotide sequence is labeled with a second moiety selected from the group consisting of said donor moiety and said acceptor moiety, and said second moiety is the member of said group not labeling said first nucleotide sequence, wherein said fourth nucleotide sequence is sufficiently complementary in reverse order to said second nucleotide sequence for a duplex to form between said fourth nucleotide sequence and said second nucleotide sequence such that said first moiety and second moiety are in sufficient proximity such that, when the donor moiety is excited and emits energy, the acceptor moiety absorbs energy emitted by the donor moiety; and wherein when said duplex is not formed, said first moiety and said second moiety are separated by a distance that prevents molecular energy transfer between said first and second moiety. In embodiments where a non-nucleotide spacer is employed in place of a nucleotide sequence, the spacer can be any linking moiety or series of linking moieties that provide a spacing equivalent to 3-20 nucleotides between the second and third nucleotide sequences and permit the hairpin primer to function as described herein. Suitable spacers are known to those of skill in the art. For example, Glen Research sells 9-O-Dimethoxytrityl-triethylene glycol,1-[(2-cyanoethyl)-(N,N-diisopropyl)]-phosphoramidite (C₃₆H₄₉N₂O₇P), which can be employed as a spacer in the hairpin primers described herein.

When the hairpin primer is used as a forward primer in the method described herein, the portion of the first nucleotide sequence that is sufficiently complementary to one or more target sequence(s) includes a sequence specific for the RNA molecule to be detected. In some embodiments, when the hairpin primer is used as a reverse primer in the method described herein, the portion of the first nucleotide sequence that is sufficiently complementary to one or more target sequence(s) includes a sequence specific for the RNA molecule to be detected. In some embodiments, when the hairpin primer is used as a reverse primer in the method, the portion of the first nucleotide sequence that is sufficiently complementary to one or more target sequence(s) includes sequence that anneals to poly(ribonucleotide) sequence (e.g., an oligo(dT) sequence or an oligo(dA) sequence). In some embodiments, the sequence that anneals to the poly(ribonucleotide) sequence ranges from 7 to 20 or 7 to 15 or 8, 9, 10, 11, 12, 13, or 14 nucleotides in length (or has a length within a range bounded by any of these values). In some embodiments, when the hairpin primer is used as a reverse primer in the method described herein, the hairpin primer includes a sequence that anneals to a poly(ribonucleotide) sequence and, at the 3' end of this sequence (i.e., at the 3' end of the primer), a sequence specific for the RNA molecule to be detected. In some embodiments, this target-specific sequence ranges from 2 to 10 or 3, 4, 5, 6, 7, 8, or 9 nucleotides in length (or has a length within a range bounded by any of these values).

The donor and acceptor moieties can be located on either terminal nucleotides of the hairpin stem (duplex region), or internally located. In some embodiments, donor and acceptor moieties are attached to the complementary strands of the stem, one moiety on the 5' end and the other moiety 5 bp apart on the complementary strand. For example, the two moieties can be offset by a 5 bp (180 degree) turn of the double helix formed by the two complementary strands of the stem, and will therefore be in closest proximity sterically, and the emission of the donor will be transferred to (and, e.g., quenched by) the acceptor. In some embodiments, the two moieties can be on complementary strands of the stem separated by a distance of less than 1 nucleotide (3.4 Angstroms) when the hairpin is in the closed configuration. In some embodiments, the two moieties are on complementary nucleotides on the stem, directly opposite from one another when the hairpin is in the closed configuration.

When a hairpin primer is linearized, the donor moiety must be separated from the acceptor (e.g., quencher) moiety by an intervening sequence that is long enough to substantially prevent MET. Where a FRET pair that consists of donor and acceptor fluorophores is used, the two FRET moieties are separated by an intervening sequence, comprising (a) at least a portion of the first stem sequence, (b) the loop, and (c) at least a portion of the second stem sequence. In some embodiments, the intervening sequence is 15-25 nucleotides in length, and in some embodiments, 20 nucleotides in length.

In some embodiments, the acceptor moiety is a fluorophore that will re-emit the energy provided by the donor at a different wavelength; that is, when the primer is in the closed state, emissions from the acceptor, but not from the donor, will be detected. In some embodiments, the acceptor moiety is a quencher and absorbs the energy emitted by the donor without fluorescing. In either case, the fluorescence of donor may be detected only when the primer is in the linearized, open state i.e., is incorporated into a double-stranded amplification product. Energy transfer in this state will be minimal and the strong emission signal from the donor will be detected.

Primers may be synthesized by standard methods known in the art, e.g., by use of an automated DNA synthesizer (such as are commercially available from Biosearch, Applied Biosystems, etc.). As examples, phosphorothioate primers may be synthesized by the method of Stein et al. (1988, Nucl. Acids Res. 16:3209), methylphosphonate primers can be prepared by use of controlled pore glass polymer supports (Sarin et al., 1988, Proc. Natl. Acad. Sci. U.S.A. 85:7448-7451), etc.

Primers may be labeled with donor and acceptor moieties during chemical synthesis or the label may be attached after synthesis by methods known in the art. In some embodiments, the donor moiety is a fluorophore. In some embodiments, both donor and acceptor moieties are fluorophores.

One of ordinary skill in the art can easily determine, using art-known techniques of spectrophotometry, which fluorophores will make suitable donor-acceptor FRET pairs. For example, FAM (which has an emission maximum of 520 nm) is a suitable donor for TAMRA, ROX, and R6G (all of which have an excitation maximum of 514 nm) in a FRET pair. Primers are preferably modified during synthesis, such that a modified dT-base is introduced into a designated position by the use of Amino-Modifier C6 dT (Glen Research), and a primary amino group is incorporated on the modified dT-base, as described by Ju et al. (1995, Proc. Natl. Acad. Sci., USA 92:4347-4351). These modifications may be used for subsequent incorporation of fluorescent dyes into designated positions of the primers.

### Exemplary Automation and Systems

In some embodiments, a target gene is detected using an automated sample handling and/or analysis platform. In some embodiments, commercially available automated analysis platforms are utilized. For example, in some embodiments, the GeneXpert^{®} system (Cepheid, Sunnyvale, CA) is utilized.

The methods described herein are illustrated for use with the GeneXpert system. Exemplary sample preparation and analysis methods are described below. However, the present invention is not limited to a particular detection method or analysis platform. One of skill in the art recognizes that any number of platforms and methods may be utilized.

The GeneXpert^{®} utilizes a self-contained, single use cartridge. Sample extraction, amplification, and detection may all be carried out within this self-contained "laboratory in a cartridge." (See e.g., US Patents 5,958,349, 6,403,037, 6,440,725, 6,783,736, 6,818,185).

Components of the cartridge include, but are not limited to, processing chambers containing reagents, filters, and capture technologies useful to extract, purify, and amplify target nucleic acids. A valve enables fluid transfer from chamber to chamber and contains nucleic acids lysis and filtration components. An optical window enables real-time optical detection. A reaction tube enables very rapid thermal cycling.

In some embodiments, the GenXpert^{®} system includes a plurality of modules for scalability. Each module includes a plurality of cartridges, along with sample handling and analysis components.

After the sample is added to the cartridge, the sample is contacted with lysis buffer and released nucleic acid is bound to a nucleic acid-binding substrate such as a silica or glass substrate. The sample supernatant is then removed and the nucleic acid eluted in an elution buffer such as a Tris/EDTA buffer. The eluate may then be processed in the cartridge to detect target genes as described herein. In some embodiments, the eluate is used to reconstitute at least some of the reagents, which are present in the cartridge as lyophilized particles.

In some embodiments, PCR is used to amplify and detect the presence of one or more RNA molecule(s). In some embodiments, the PCR uses Taq polymerase with hot start function, such as AptaTaq (Roche).

In some embodiments, an off-line centrifugation is used to improve assay results with samples with low cellular content. The sample, with or without the buffer added, is centrifuged and the supernatant removed. The pellet is then resuspended in a smaller volume of supernatant, buffer, or other liquid. The resuspended pellet is then added to a GeneXpert^{®} cartridge as previously described.

### Kits

Also a kit for carrying out the methods is described herein. Such kits include one or more reagents useful for practicing any of these methods. A kit generally includes a package with one or more containers holding the reagents, as one or more separate compositions or, optionally, as an admixture where the compatibility of the reagents will allow. The kit can also include other material(s) that may be desirable from a user standpoint, such as a buffer(s), a diluent(s), a standard(s), and/or any other material useful in sample processing, washing, or conducting any other step of the assay.

Kits preferably include instructions for carrying out one or more of the screening methods described herein. Instructions included in kits of the invention can be affixed to packaging material or can be included as a package insert. While the instructions are typically written or printed materials they are not limited to such. Any medium capable of storing such instructions and communicating them to an end user is contemplated by this invention. Such media include, but are not limited to, electronic storage media (e.g., magnetic discs, tapes, cartridges, chips), optical media (e.g., CD ROM), and the like. As used herein, the term "instructions" can include the address of an internet site that provides the instructions.

In some aspects of the disclosure, a kit includes: (a) a forward primer and a reverse primer, as described above, wherein the forward primer is specific for the RNA molecule to be detected, and the reverse primer includes a sequence that anneals to a poly(ribonucleotide) sequence; wherein at least one of the forward and reverse primers includes a hairpin primer, as described above; and (b) an enzyme capable of adding the poly(ribonucleotide) sequence to RNA molecules.

In some aspects of the disclosure, a kit includes a forward primer and a reverse primer, as described above, wherein the forward primer is specific for the RNA molecule to be detected, and the reverse primer includes a sequence that anneals to a poly(ribonucleotide) sequence; wherein at least one of the forward and reverse primers includes a hairpin primer, as described above; wherein the kit includes no additional primers or probes for amplifying and detecting cDNA molecules corresponding to said one or more specific RNA molecule(s). In some aspects of the disclosure, this kit includes an enzyme capable of adding the poly(ribonucleotide) sequence to RNA molecules.

In some aspects of the disclosureof these kits, the reverse primer additionally includes a nucleotide sequence specific for the RNA molecule to be detected at the 3' end of the poly(ribonucleotide) sequence.

In some aspects of the disclosureof these kits, the poly(ribonucleotide) sequence is a poly(A) sequence, and the sequence that anneals to this poly(A) sequence includes an oligo(dT) sequence. In some aspects of the disclosure, such kits include a poly(A)polymerase to add the poly(A) sequence to RNA molecules.

In some aspects of the disclosureof these kits, the poly(ribonucleotide) sequence is a poly(U) sequence, and the sequence that anneals to this poly(U) sequence includes an oligo(dA) sequence. In some aspects of the disclosure, such kits include a uridylyl transferase or a poly(U)polymerase to add the poly(U) sequence to RNA molecules.

In some aspects of the disclosure, the forward primer includes a hairpin primer and the reverse primer does not include a hairpin primer. In some aspects of the disclosure, the reverse primer includes a hairpin primer and the forward primer does not include a hairpin primer.

In some aspects of the disclosure, the kit includes a reverse transcriptase. In some aspects of the disclosure, the reverse transcriptase is provided in the same container as the enzyme capable of adding the poly(ribonucleotide) sequence to RNA molecules. In some aspects of the disclosure, the kit includes a DNA polymerase. In some aspects of the disclosure, a single enzyme serves as the reverse transcriptase and the DNA polymerase. In some aspects of the disclosure, the kit includes enzyme capable of adding the poly(ribonucleotide) sequence to RNA molecules together with a reverse transcriptase and a DNA polymerase in the form of a single enzyme. In some aspects of the disclosure, these two enzymes are provided in one container.

In some embodiments useful for multiplex methods, the kit includes at least two different primer pairs, each of which is specific for one RNA molecule to be detected. In some embodiments, at least one primer pair includes a hairpin primer, more than one primer pair includes a hairpin primer, or all primer pairs in the reaction include a hairpin primer. In some embodiments, each hairpin primer includes a means for emitting a detectable signal when the strands of the duplex are separated that is distinguishable from all others in a reaction. In some embodiments, the kit includes at least 3, 4, 5, 6, 7, 8, 9, or 10 different primer pairs, each of which is specific for one RNA molecule to be detected.

In some embodiments, the kit includes one or more primer pair(s) for detecting a non-coding RNA, as described above. In some embodiments, the kit additionally includes one or more primer pair(s) for amplifying one or more mRNA molecule(s) with the non-coding RNA in a multiplex reaction. In some embodiments, the primer pair(s) for amplifying one or more mRNA molecule(s) include(s) a hairpin primer. In some embodiments, the primer pair(s) for amplifying one or more mRNA molecule(s) do/does not include(s) a hairpin primer. Where hairpin primers are not used for detection of the mRNA molecule(s), the kit can additionally include includes one or more probe(s) for detecting the mRNA molecules. In some embodiments, probes are specific for the mRNA molecules.

It is understood that the examples and embodiments described herein are for illustrative purposes only.

### EXAMPLES

### Example 1: Multiplex miRNA Assay

A multiplex miRNA assay was carried as described above using two primer pairs in which the reverse primers were hairpin primers. Each reverse primer include a oligo(dT) sequence and a 5-base target-specific sequence 3' of the oligo(dT) sequence. The targets were miR31 and CTL23. The primers used are shown in Table 1.

**Table 1: oligonucleotides**

| oligo # | oligo name | 5' | | 3' |
|---|---|---|---|---|
| 13295 | miR31-RT-FAM H | FAM | CGT GCG CGA AGC GCA CGG G(U01)C TCG ACT CGA CAG TTT TTT TTT TAG CT (SEQ ID NO:20) | |
| 13282 | miR31-F | | AAA CAG GCA AGA TGC TGG CAT (SEQ ID NO:21) | |
| 13969 | CTL23-2-RT-CF4-1 | CF4-CL4 | CGC ACG CGA AGC GTG CGG G(U01)C AGG CGA CAA CTC TTT TTT TTT TCA GT  (SEQ ID NO:22) | |
| 13970 | CTL23-2-F-1 | | AAACGAACTT(A01)CT(A01)TGT(A01)GGCA (SEQ ID NO:23) | |

| | | | | |
|---|---|---|---|---|
| Note: "U01" is a dT-Dabcyl conjugate (commercially available from Glen Research); "A01" is an analog of dA with increased hybridization potential to dT (2,6-diaminopurine). | | | | |

Polyadenylation and reverse transcription was carried out as follows:

**Table 2: Poly(A) plus RT formulation and assay conditions**

| | Concentration | volume(ul) |
|---|---|---|
| Poly(A) polymerase buffer (NEB) | 10x | 2 |
| ATP | 1mM | 2 |
| dNTP mix | 1 mM | 2 |
| MuLV RT (NEB) | 200U/u1 | 1 |
| Poly(A) polymerase (NEB) | 50U/u1 | 0.4 |
| RT primer 1 | 10uM | 2 |
| RT primer 2 | 10uM | 2 |
| RNA sample | | 2 |

| | | |
|---|---|---|
| RT: 40C-15 min, 90 C-4 min | | |

Quantitative real-time PCR was carried out as follows:

**Table 3: PCR formulation and assay conditions**

| PCR, 25 uL | Ix |
|---|---|
| dNTP (10mM) | 2.5 |
| Taq polymerase (5U/u1) | 0.4 |
| PCR buffer (10x) | 2.5 |
| Forward primer 1 10uM | 0.5 |
| Forward primer 2 10uM | 0.5 |
| RT primer 1 10uM | 0.5 |
| RT primer 2 10uM | 0.5 |
| cDNA | 2.5 |
| H2O | 15.1 |
| total | 25 |

| | |
|---|---|
| 93C-6 sec; 60C-35 sec'; 93C-6; sec 64C-35 sec | |

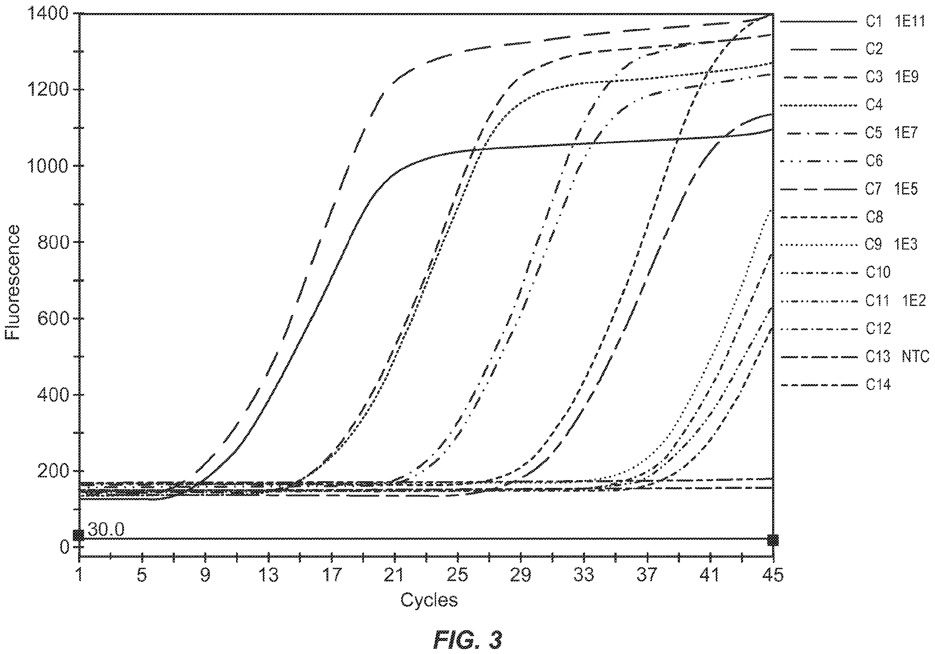

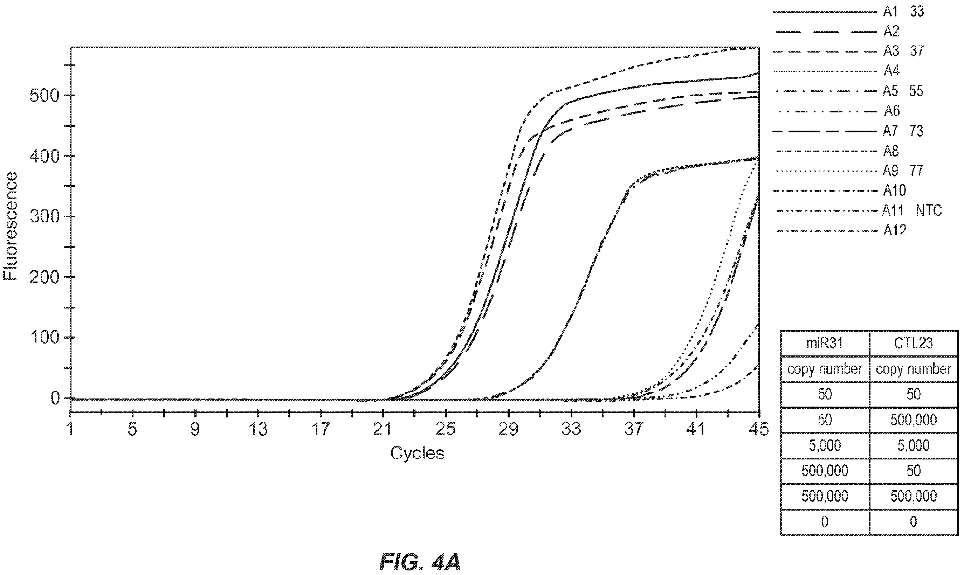

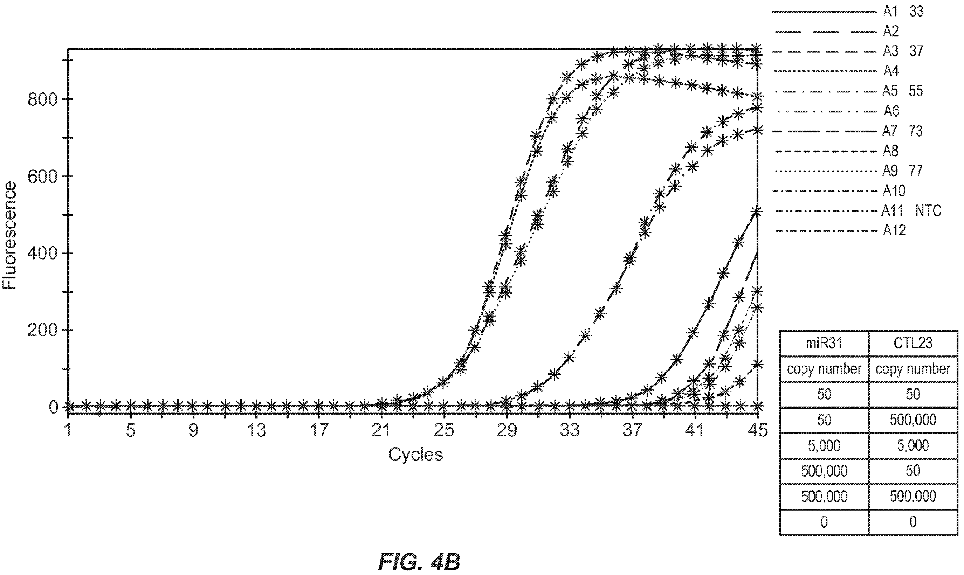

The results are shown in Figs. 3 and 4. In these graphs, fluorescence is plotted against PCR cycle number. Figure 3 shows the results of quantitative real-time PCR microRNA assays carried in singleplex with a dilution series of target. Figure 4A-B shows the results of a multiplex quantitative real-time PCR microRNA assay. These results demonstrate that miR31 (A) and CTL23 (B) can be measured in multiplex.

## Claims

1. A kit for amplifying and detecting at least two non-coding RNA molecules, the kit comprising:
(a) at least two different primer pairs, each of which is specific for one of the at least two non-coding RNA molecules in a multiplex reaction, each primer pair comprising a forward primer and a reverse primer, wherein the forward primer of each primer pair is specific for one of the at least two non-coding RNA molecules to be detected, and the reverse primer of each primer pair comprises a sequence that anneals to a poly(ribonucleotide) sequence present in or added to the non-coding RNA and a sequence specific for one of the at least two non-coding RNA molecules to be detected at the 3' end of the sequence that anneals to said poly(ribonucleotide) sequence, wherein the sequence that anneals to said poly(ribonucleotide) sequence optionally comprises an oligo(dT) sequence or an oligo(dA) sequence; wherein at least one of the forward and reverse primers of each primer pair comprises a hairpin primer comprising:
(i) a first nucleotide sequence comprising:
a sequence specific for the non-coding RNA molecule to be detected when the hairpin primer is the forward primer, or
the sequence specific for the non-coding RNA molecule to be detected at the 3' end of the sequence that anneals to said poly(ribonucleotide) sequence when the hairpin primer is the reverse primer;
(ii) a second nucleotide sequence at the 5' end of the first sequence;
(iii) a third nucleotide sequence or spacer at the 5' end of the second sequence;
(iv) a fourth nucleotide sequence at the 5' end of the third sequence or spacer, the fourth sequence being complementary to the second sequence, so as to form a double-stranded duplex; and
(v) means for emitting a detectable signal when the strands of the duplex are separated, wherein each hairpin primer in the kit produces a different detectable signal; and
(b) wherein the kit either comprises an enzyme capable of adding said poly(ribonucleotide) sequence to RNA molecules or the kit comprises no additional primers or probes for amplifying and detecting cDNA molecules corresponding to said non-coding RNA molecules to be detected.

2. The kit of claim 1, wherein for each primer pair, the forward primer comprises a hairpin primer and the reverse primer does not comprise a hairpin primer.

3. The kit of claim 1, wherein for each primer pair, the reverse primer comprises a hairpin primer and the forward primer does not comprise a hairpin primer.

4. The kit of any of claims 1-3, wherein the kit comprises an enzyme capable of adding said poly(ribonucleotide) sequence to RNA.

5. The kit of any of claims 1-4, wherein the enzyme capable of adding said poly(ribonucleotide) sequence to RNA molecules comprises a poly(A)polymerase, a uridylyl transferase, or a poly(U)polymerase.

6. The kit of any of claims 1-5, wherein the kit comprises at least 3, 4, 5, 6, 7, 8, 9, or 10 different primer pairs, each of which is specific for one non-coding RNA molecule to be detected, wherein each hairpin primer in the kit produces a different detectable signal.

7. The kit of any of claims 1-6, wherein for each primer pair, the signal-emitting means comprises an energy donor moiety and an energy acceptor moiety, each bound to the hairpin primer and spaced such that the signal is detectable only when the strands of the duplex are separated, optionally wherein (a) the energy donor and acceptor moieties are spaced a distance in the range of about 10-40 nucleotides, (b) the acceptor moiety is a fluorophore that emits fluorescent light at a wavelength different than that emitted by the donor moiety, (c) the energy donor moiety is a fluorophore and the energy acceptor moiety is a fluorophore quencher, or (d) the donor moiety is selected from the group consisting of fluorescein, 5-carboxyfluorescein (FAM), rhodamine, 5-(2'-aminoethyl) aminonapthalene-1-sulfonic acid (EDANS), anthranilamide, coumarin, terbium chelate derivatives, and Reactive Red 4, and the acceptor moiety is selected from the group consisting of DABCYL, rhodamine, tetramethyl rhodamine, pyrene butyrate, eosine nitrotyrosine, ethidium, fluorescein, Malachite green, and Texas Red.

8. The kit of claim 7, wherein for each primer pair, the energy donor moiety and the energy acceptor moiety are situated on opposite strand nucleotides that are five, or fewer, nucleotides apart in the duplex, preferably wherein the energy donor moiety and the energy acceptor moiety are situated on complementary nucleotides that are opposite each other in the duplex.

9. The kit of any of claims 1-8, wherein the sequence that anneals to said poly(ribonucleotide) sequence comprises an oligo(dT) sequence or an oligo(dA) sequence.

10. The kit of any of claims 1-9, wherein the sequence specific for the non-coding RNA molecule to be detected comprises at least four bases.

11. A method for amplifying and detecting at least two non-coding RNA molecules from a sample in a multiplex reaction, the method comprising:
(a) adding a poly(ribonucleotide) sequence to non-coding RNA molecules in the sample to produce poly(ribonucleotide)-non-coding RNA molecules;
(b) reverse transcribing at least two poly(ribonucleotide)-non-coding RNA molecule(s) to be detected using at least two reverse primers that are each specific for one of the non-coding RNA molecules to be detected, wherein each reverse primer comprises a sequence that anneals to said poly(ribonucleotide) sequence and a sequence specific for one of the at least two non-coding RNA molecules to be detected at the 3' end of the sequence that anneals to said poly(ribonucleotide) sequence, to produce at least two cDNA molecules; and
(c) amplifying and detecting each cDNA molecule using said reverse primers and using a forward primer specific for the non-coding RNA molecule to be detected;
wherein at least one of the forward and reverse primers for each non-coding RNA to be detected comprises a hairpin primer comprising:
(i) a first nucleotide sequence comprising:
a sequence specific for the non-coding RNA molecule to be detected when the hairpin primer is the forward primer, or
the sequence specific for the non-coding RNA molecule to be detected at the 3' end of the sequence that anneals to said poly(ribonucleotide) sequence when the hairpin primer is the reverse primer;
(ii) a second nucleotide sequence at the 5' end of the first sequence;
(iii) a third nucleotide sequence or spacer at the 5' end of the second sequence;
(iv) a fourth nucleotide sequence at the 5' end of the third sequence or spacer, the fourth sequence being complementary to the second sequence, so as to form a double-stranded duplex; and
(v) means for emitting a detectable signal when the strands of the duplex are separated, wherein the hairpin primer produces a detectable signal when the non-coding RNA to be detected has been amplified.

12. The method of claim 11, wherein the sequence that anneals to said poly(ribonucleotide) sequence comprises an oligo(dT) sequence or an oligo(dA) sequence.

13. The method of claim 11 or claim 12, wherein the sequence specific for the non-coding RNA molecule to be detected comprises at least four bases.

14. The method of any of claims 11-13, wherein for each primer pair, the forward primer comprises a hairpin primer and the reverse primer does not comprise a hairpin primer.

15. The method of any of claims 11-13, wherein for each primer pair, the reverse primer comprises a hairpin primer and the forward primer does not comprise a hairpin primer.

16. The method of any of claims 11-15, wherein the at least two non-coding RNA molecules detected comprise at least 3, 4, 5, 6, 7, 8, 9, or 10 different non-coding RNAs.

## Patentansprüche

1. Kit zum Amplifizieren und Nachweisen von mindestens zwei nicht kodierenden RNA-Molekülen, wobei das Kit umfasst:
(a) mindestens zwei unterschiedliche Primerpaare, von denen jedes für eines der mindestens zwei nicht kodierenden RNA-Moleküle in einer Multiplex-Reaktion spezifisch ist, wobei jedes Primerpaar einen Vorwärtsprimer und einen Rückwärtsprimer umfasst, wobei der Vorwärtsprimer jedes Primerpaares für eines der mindestens zwei nachzuweisenden nicht kodierenden RNA-Moleküle spezifisch ist und der Rückwärtsprimer jedes Primerpaares eine Sequenz, die sich an eine Poly(ribonukleotid)-Sequenz anlagert, die in der nicht kodierenden RNA vorhanden ist oder zu der nicht kodierenden RNA hinzugefügt wurde, und eine Sequenz umfasst, die für eines der mindestens zwei nachzuweisenden nicht kodierenden RNA-Moleküle an dem 3'-Ende der Sequenz spezifisch ist und die sich an die Poly(ribonukleotid)-Sequenz anlagert, wobei die Sequenz, die sich an die Poly(ribonukleotid)-Sequenz anlagert, optional eine Oligo(dT)-Sequenz oder eine Oligo(dA)-Sequenz umfasst; wobei mindestens einer der Vorwärts- und Rückwärtsprimer jedes Primerpaares einen Haarnadelprimer umfasst, umfassend:
(i) eine erste Nukleotidsequenz, umfassend:
eine Sequenz, die für das nachzuweisende nicht kodierende RNA-Molekül spezifisch ist, wenn der Haarnadelprimer der Vorwärtsprimer ist, oder
die Sequenz, die für das nachzuweisende nicht kodierende RNA-Molekül an dem 3'-Ende der Sequenz spezifisch ist und die sich an die Poly(ribonukleotid)-Sequenz anlagert, wenn der Haarnadelprimer der Rückwärtsprimer ist;
(ii) eine zweite Nukleotidsequenz an dem 5'-Ende der ersten Sequenz;
(iii) eine dritte Nukleotidsequenz oder einen Spacer an dem 5'-Ende der zweiten Sequenz;
(iv) eine vierte Nukleotidsequenz an dem 5'-Ende der dritten Sequenz oder des Spacers, wobei die vierte Sequenz zu der zweiten Sequenz komplementär ist, um ein doppelsträngiges Duplex zu bilden; und
(v) Mittel zum Emittieren eines nachweisbaren Signals, wenn die Stränge des Duplex getrennt sind, wobei jeder Haarnadelprimer in dem Kit ein unterschiedliches nachweisbares Signal erzeugt; und
(b) wobei das Kit entweder ein Enzym umfasst, das in der Lage ist, die Poly(ribonukleotid)-Sequenz zu RNA-Molekülen hinzuzufügen, oder das Kit keine zusätzlichen Primer oder Sonden zum Amplifizieren und Nachweisen von cDNA-Molekülen umfasst, die den nachzuweisenden nicht kodierenden RNA-Molekülen entsprechen.

2. Kit nach Anspruch 1, wobei für jedes Primerpaar der Vorwärtsprimer einen Haarnadelprimer umfasst und der Rückwärtsprimer keinen Haarnadelprimer umfasst.

3. Kit nach Anspruch 1, wobei für jedes Primerpaar der Rückwärtsprimer einen Haarnadelprimer umfasst und der Vorwärtsprimer keinen Haarnadelprimer umfasst.

4. Kit nach einem der Ansprüche 1-3, wobei das Kit ein Enzym umfasst, das in der Lage ist, die Poly(ribonukleotid)-Sequenz zu RNA hinzuzufügen.

5. Kit nach einem der Ansprüche 1-4, wobei das Enzym, das in der Lage ist, die Poly(ribonukleotid)-Sequenz zu RNA-Molekülen hinzuzufügen, eine Poly(A)polymerase, eine Uridylyltransferase oder eine Poly(U)polymerase umfasst.

6. Kit nach einem der Ansprüche 1-5, wobei das Kit mindestens 3, 4, 5, 6, 7, 8, 9 oder 10 unterschiedliche Primerpaare umfasst, von denen jedes für ein nachzuweisendes nicht kodierendes RNA-Molekül spezifisch ist, wobei jeder Haarnadelprimer in dem Kit ein unterschiedliches nachweisbares Signal erzeugt.

7. Kit nach einem der Ansprüche 1-6, wobei für jedes Primerpaar das signalemittierende Mittel einen Energiedonorrest und einen Energieakzeptorrest umfasst, die jeweils an den Haarnadelprimer gebunden und so beabstandet sind, dass das Signal nur nachweisbar ist, wenn die Stränge des Duplex getrennt sind, wobei optional (a) die Energiedonor- und -akzeptorreste um einen Abstand im Bereich von etwa 10-40 Nukleotiden beabstandet sind, (b) der Akzeptorrest ein Fluorophor ist, das Fluoreszenzlicht bei einer Wellenlänge emittiert, die sich von der unterscheidet, die von dem Donorrest emittiert wird, (c) der Energiedonorrest ein Fluorophor ist und der Energieakzeptorrest ein Fluorophorquencher ist oder (d) der Donorrest ausgewählt ist aus der Gruppe bestehend aus Fluoreszein, 5-Carboxyfluoreszein (FAM), Rhodamin, 5-(2'-Aminoethyl)aminonaphthalin-1-sulfonsäure (EDANS), Anthranilamid, Kumarin, Terbiumchelat-Derivaten und Reactive Red 4, und der Akzeptorrest ausgewählt ist aus der Gruppe bestehend aus DABCYL, Rhodamin, Tetramethylrhodamin, Pyrenbutyrat, Eosinnitrotyrosin, Ethidium, Fluoreszein, Malachitgrün und Texas Red.

8. Kit nach Anspruch 7, wobei für jedes Primerpaar der Energiedonorrest und der Energieakzeptorrest an gegenüberliegenden Strangnukleotiden angeordnet sind, die in dem Duplex um fünf oder weniger Nukleotide beabstandet sind, wobei vorzugsweise der Energiedonorrest und der Energieakzeptorrest auf komplementären Nukleotiden angeordnet sind, die einander im Duplex gegenüberliegen.

9. Kit nach einem der Ansprüche 1-8, wobei die Sequenz, die sich an die Poly(ribonukleotid)-Sequenz anlagert, eine Oligo(dT)-Sequenz oder eine Oligo(dA)-Sequenz umfasst.

10. Kit nach einem der Ansprüche 1-9, wobei die Sequenz, die für das nachzuweisende nicht kodierende RNA-Molekül spezifisch ist, mindestens vier Basen umfasst.

11. Verfahren zum Amplifizieren und Nachweisen von mindestens zwei nicht kodierenden RNA-Molekülen aus einer Probe in einer Multiplex-Reaktion, wobei das Verfahren umfasst:
(a) Hinzufügen einer Poly(ribonukleotid)-Sequenz zu nicht kodierenden RNA-Molekülen in der Probe, um Poly(ribonukleotid)-nicht-kodierende-RNA-Moleküle zu erzeugen;
(b) reverses Transkribieren von mindestens zwei nachzuweisenden Poly(ribonukleotid)-nicht-kodierenden-RNA-Moleküle(n) unter Verwendung von mindestens zwei Rückwärtsprimern, die jeweils für eines der nachzuweisenden nicht kodierenden RNA-Moleküle spezifisch sind, wobei jeder Rückwärtsprimer eine Sequenz umfasst, die sich an die Poly(ribonukleotid)-Sequenz anlagert, und eine Sequenz, die für eines der mindestens zwei nachzuweisenden nicht kodierenden RNA-Moleküle an dem 3'-Ende der Sequenz spezifisch ist und sich an die Poly(ribonukleotid)-Sequenz anlagert, um mindestens zwei cDNA-Moleküle zu erzeugen; und
(c) Amplifizieren und Nachweisen jedes cDNA-Moleküls unter Verwendung der Rückwärtsprimer und unter Verwendung eines Vorwärtsprimers, der für das nachzuweisende nicht kodierende RNA-Molekül spezifisch ist;
wobei mindestens einer der Vorwärts- und Rückwärtsprimer für jede nachzuweisende nicht kodierende RNA einen Haarnadelprimer umfasst, umfassend:
(i) eine erste Nukleotidsequenz, umfassend:
eine Sequenz, die für das nachzuweisende nicht kodierende RNA-Molekül spezifisch ist, wenn der Haarnadelprimer der Vorwärtsprimer ist, oder
die Sequenz, die für das nachzuweisende nicht kodierende RNA-Molekül an dem 3'-Ende der Sequenz spezifisch ist und die sich an die Poly(ribonukleotid)-Sequenz anlagert, wenn der Haarnadelprimer der Rückwärtsprimer ist;
(ii) eine zweite Nukleotidsequenz an dem 5'-Ende der ersten Sequenz;
(iii) eine dritte Nukleotidsequenz oder einen Spacer an dem 5'-Ende der zweiten Sequenz;
(iv) eine vierte Nukleotidsequenz an dem 5'-Ende der dritten Sequenz oder des Spacers, wobei die vierte Sequenz zu der zweiten Sequenz komplementär ist, um ein doppelsträngiges Duplex zu bilden; und
(v) Mittel zum Emittieren eines nachweisbaren Signals, wenn die Stränge des Duplex getrennt sind, wobei der Haarnadelprimer ein nachweisbares Signal erzeugt, wenn die nachzuweisende nicht kodierende RNA amplifiziert wurde.

12. Verfahren nach Anspruch 11, wobei die Sequenz, die sich an die Poly(ribonukleotid)-Sequenz anlagert, eine Oligo(dT)-Sequenz oder eine Oligo(dA)-Sequenz umfasst.

13. Verfahren nach Anspruch 11 oder Anspruch 12, wobei die Sequenz, die für das nachzuweisende nicht kodierende RNA-Molekül spezifisch ist, mindestens vier Basen umfasst.

14. Verfahren nach einem der Ansprüche 11-13, wobei für jedes Primerpaar der Vorwärtsprimer einen Haarnadelprimer umfasst und der Rückwärtsprimer keinen Haarnadelprimer umfasst.

15. Verfahren nach einem der Ansprüche 11-13, wobei für jedes Primerpaar der Rückwärtsprimer einen Haarnadelprimer umfasst und der Vorwärtsprimer keinen Haarnadelprimer umfasst.

16. Verfahren nach einem der Ansprüche 11-15, wobei die mindestens zwei nachgewiesenen nicht kodierenden RNA-Moleküle mindestens 3, 4, 5, 6, 7, 8, 9 oder 10 unterschiedliche nicht kodierende RNAs umfassen.

## Revendications

1. Trousse d'amplification et de détection d'au moins deux molécules d'ARN non codant, la trousse comprenant :
(a) au moins deux paires d'amorces différentes, dont chacune est spécifique de l'une des au moins deux molécules d'ARN non codant dans une réaction multiplexe, chaque paire d'amorces comprenant une amorce sens et une amorce antisens, dans laquelle l'amorce sens de chaque paire d'amorces est spécifique de l'une des au moins deux molécules d'ARN non codant à détecter, et l'amorce antisens de chaque paire d'amorces comprend une séquence qui s'hybride à une séquence poly(ribonucléotidique) présente dans ou ajoutée à l'ARN non codant et une séquence spécifique de l'une des au moins deux molécules d'ARN non codant à détecter à l'extrémité 3' de la séquence qui s'hybride à ladite séquence poly(ribonucléotidique), dans laquelle la séquence qui s'hybride à ladite séquence poly(ribonucléotidique) comprend facultativement une séquence oligo(dT) ou une séquence oligo(dA) ; dans laquelle au moins l'une des amorces sens et antisens de chaque paire d'amorces comprend une amorce en épingle à cheveux comprenant :
(i) une première séquence nucléotidique comprenant :
une séquence spécifique de la molécule d'ARN non codant à détecter lorsque l'amorce en épingle à cheveux est l'amorce sens, ou
la séquence spécifique de la molécule d'ARN non codant à détecter à l'extrémité 3' de la séquence qui s'hybride à ladite séquence poly(ribonucléotidique) lorsque l'amorce en épingle à cheveux est l'amorce antisens ;
(ii) une deuxième séquence nucléotidique à l'extrémité 5' de la première séquence ;
(iii) une troisième séquence nucléotidique ou un espaceur à l'extrémité 5' de la deuxième séquence ;
(iv) une quatrième séquence nucléotidique à l'extrémité 5' de la troisième séquence ou de l'espaceur, la quatrième séquence étant complémentaire de la deuxième séquence, de manière à former un duplex double brin ; et
(v) un moyen d'émission d'un signal détectable lorsque les brins du duplex sont séparés, dans laquelle chaque amorce en épingle à cheveux dans la trousse produit un signal détectable différent ; et
(b) dans laquelle la trousse comprend soit une enzyme capable d'ajouter ladite séquence poly(ribonucléotidique) à des molécules d'ARN soit la trousse ne comprend pas d'amorces ou de sondes supplémentaires d'amplification et de détection de molécules d'ADNc correspondant auxdites molécules d'ARN non codant à détecter.

2. Trousse selon la revendication 1, dans laquelle pour chaque paire d'amorces, l'amorce sens comprend une amorce en épingle à cheveux et l'amorce antisens ne comprend pas d'amorce en épingle à cheveux.

3. Trousse selon la revendication 1, dans laquelle pour chaque paire d'amorces, l'amorce antisens comprend une amorce en épingle à cheveux et l'amorce sens ne comprend pas d'amorce en épingle à cheveux.

4. Trousse selon l'une quelconque des revendications 1 à 3, dans laquelle la trousse comprend une enzyme capable d'ajouter ladite séquence poly(ribonucléotidique) à l'ARN.

5. Trousse selon l'une quelconque des revendications 1 à 4, dans laquelle l'enzyme capable d'ajouter ladite séquence poly(ribonucléotidique) à des molécules d'ARN comprend une poly(A)polymérase, une uridylyltransférase, ou une poly(U)polymérase.

6. Trousse selon l'une quelconque des revendications 1 à 5, dans laquelle la trousse comprend au moins 3, 4, 5, 6, 7, 8, 9 ou 10 paires d'amorces différentes, dont chacune est spécifique d'une molécule d'ARN non codant à détecter, dans laquelle chaque amorce en épingle à cheveux dans la trousse produit un signal détectable différent.

7. Trousse selon l'une quelconque des revendications 1 à 6, dans laquelle pour chaque paire d'amorces, le moyen d'émission de signal comprend un fragment donneur d'énergie et un fragment accepteur d'énergie, chacun lié à l'amorce en épingle à cheveux et espacé de telle sorte que le signal est détectable uniquement lorsque les brins du duplex sont séparés, facultativement dans laquelle (a) les fragments donneur et accepteur d'énergie sont espacés d'une distance comprise dans la plage d'environ 10 à 40 nucléotides, (b) le fragment accepteur est un fluorophore qui émet une lumière fluorescente à une longueur d'onde différente de celle émise par le fragment donneur, (c) le fragment donneur d'énergie est un fluorophore et le fragment accepteur d'énergie est un extincteur de fluorophore, ou (d) le fragment donneur est choisi dans le groupe constitué par la fluorescéine, la 5-carboxyfluorescéine (FAM), la rhodamine, l'acide 5-(2'-aminoéthyl)aminonapthalène-1-sulfonique (EDANS), l'anthranilamide, la coumarine, les dérivés chélates de terbium et le Reactive Red 4, et le fragment accepteur est choisi dans le groupe constitué par le DABCYL, la rhodamine, la tétraméthylrhodamine, le butyrate de pyrène, l'éosine nitrotyrosine, l'éthidium, la fluorescéine, le vert de Malachite et le Texas Red.

8. Trousse selon la revendication 7, dans laquelle pour chaque paire d'amorces, le fragment donneur d'énergie et le fragment accepteur d'énergie sont situés sur des nucléotides de brins opposés qui sont séparés de cinq nucléotides, ou moins, dans le duplex, de préférence dans laquelle le fragment donneur d'énergie et le fragment accepteur d'énergie sont situés sur des nucléotides complémentaires qui sont opposés l'un à l'autre dans le duplex.

9. Trousse selon l'une quelconque des revendications 1 à 8, dans laquelle la séquence qui s'hybride à ladite séquence poly(ribonucléotidique) comprend une séquence oligo(dT) ou une séquence oligo(dA).

10. Trousse selon l'une quelconque des revendications 1 à 9, dans laquelle la séquence spécifique de la molécule d'ARN non codant à détecter comprend au moins quatre bases.

11. Procédé d'amplification et de détection d'au moins deux molécules d'ARN non codant à partir d'un échantillon dans une réaction multiplexe, le procédé comprenant :
(a) l'ajout d'une séquence poly(ribonucléotidique) à des molécules d'ARN non codant dans l'échantillon pour produire des molécules d'ARN non codant poly(ribonucléotidiques) ;
(b) la transcription inverse d'au moins deux molécules d'ARN non codant poly(ribonucléotidiques) à détecter à l'aide d'au moins deux amorces antisens qui sont chacune spécifique de l'une des molécules d'ARN non codant à détecter, dans lequel chaque amorce antisens comprend une séquence qui s'hybride à ladite séquence poly(ribonucléotidique) et une séquence spécifique de l'une des au moins deux molécules d'ARN non codant à détecter à l'extrémité 3' de la séquence qui s'hybride à ladite séquence poly(ribonucléotidique), pour produire au moins deux molécules d'ADNc ; et
(c) l'amplification et la détection de chaque molécule d'ADNc à l'aide desdites amorces antisens et à l'aide d'une amorce sens spécifique de la molécule d'ARN non codant à détecter ;
dans lequel au moins l'une des amorces sens et antisens pour chaque ARN non codant à détecter comprend une amorce en épingle à cheveux comprenant :
(i) une première séquence nucléotidique comprenant :
une séquence spécifique de la molécule d'ARN non codant à détecter lorsque l'amorce en épingle à cheveux est l'amorce directe, ou
la séquence spécifique de la molécule d'ARN non codant à détecter à l'extrémité 3' de la séquence qui s'hybride à ladite séquence poly(ribonucléotidique) lorsque l'amorce en épingle à cheveux est l'amorce antisens ;
(ii) une deuxième séquence nucléotidique à l'extrémité 5' de la première séquence ;
(iii) une troisième séquence nucléotidique ou un espaceur à l'extrémité 5' de la deuxième séquence ;
(iv) une quatrième séquence nucléotidique à l'extrémité 5' de la troisième séquence ou de l'espaceur, la quatrième séquence étant complémentaire de la deuxième séquence, de manière à former un duplex double brin ; et
(v) un moyen d'émission d'un signal détectable lorsque les brins du duplex sont séparés, dans lequel l'amorce en épingle à cheveux produit un signal détectable lorsque l'ARN non codant à détecter a été amplifié.

12. Procédé selon la revendication 11, dans lequel la séquence qui s'hybride à ladite séquence poly(ribonucléotidique) comprend une séquence oligo(dT) ou une séquence oligo(dA).

13. Procédé selon la revendication 11 ou la revendication 12, dans lequel la séquence spécifique de la molécule d'ARN non codant à détecter comprend au moins quatre bases.

14. Procédé selon l'une quelconque des revendications 11 à 13, dans lequel pour chaque paire d'amorces, l'amorce sens comprend une amorce en épingle à cheveux et l'amorce antisens ne comprend pas d'amorce en épingle à cheveux.

15. Procédé selon l'une quelconque des revendications 11 à 13, dans lequel pour chaque paire d'amorces, l'amorce antisens comprend une amorce en épingle à cheveux et l'amorce sens ne comprend pas d'amorce en épingle à cheveux.

16. Procédé selon l'une quelconque des revendications 11 à 15, dans lequel les au moins deux molécules d'ARN non codant détectées comprennent au moins 3, 4, 5, 6, 7, 8, 9 ou 10 ARN non codants différents.
